# EUROPEAN PATENT SPECIFICATION

(11) **EP 1 023 842 B1**
(45) Date of publication and mention of the grant of the patent: **27.09.2006**
(21) Application number: 00100273.2
(22) Date of filing: 19.01.2000
(51) Int. Cl.: A23L 1/22, A23L 2/39, A23L 2/64, B01J 3/00, A61K 47/38, A23G 3/00, C07H 3/00, A23L 1/0534, A61K 9/14, A23L 1/30, A23L 1/302, A61K 9/16, A61K 8/34, A61K 8/65, A61K 8/67, A61K 8/73, A61K 8/92

(54) **Edible powder material having excellent shelf stability**
Essbares Pulver mit guter Lagerbeständigkeit
Matière poudreuse comestible avec une excellente stabilité de conservation

(30) Priority: 27.01.1999 JP 1871599
(43) Date of publication of application: 02.08.2000
(73) Proprietor: T. Hasegawa Co., Ltd., Chuo-ku, Tokyo 103-8431 (JP)
(72) Inventor: Watanabe, Takayuki, c/o Technical Research Center, Kawasaki-shi, Kanagawa-ken (JP); Suzuki, Hisashi, c/o Technical Research Center, Kawasaki-shi, Kanagawa-ken (JP); Nakamura, Tetsuya, c/o Technical Research Center, Kawasaki-shi, Kanagawa-ken (JP)
(74) Representative: Albrecht, Thomas

(56) References cited:
- EP-A- 0 598 920
- PATENT ABSTRACTS OF JAPAN vol. 1998, no. 08, 30 June 1998 (1998-06-30) & JP 10 056969 A (FUJI OIL CO LTD), 3 March 1998 (1998-03-03)
- DATABASE WPI Section Ch, Week 199739 Derwent Publications Ltd., London, GB; Class D13, AN 1997-419376 XP002259690 & JP 09 187249 A (HASEGAWA CO LTD), 22 July 1997 (1997-07-22)
- DATABASE WPI Section Ch, Week 199524 Derwent Publications Ltd., London, GB; Class A97, AN 1995-183227 XP002259694 & JP 07 102279 A (SANEIGEN FFI KK), 18 April 1995 (1995-04-18)
- PATENT ABSTRACTS OF JAPAN vol. 1995, no. 07, 31 August 1995 (1995-08-31) & JP 07 107937 A (T HASEGAWA CO LTD), 25 April 1995 (1995-04-25)
- PATENT ABSTRACTS OF JAPAN vol. 1997, no. 08, 29 August 1997 (1997-08-29) & JP 09 107911 A (T HASEGAWA CO LTD), 28 April 1997 (1997-04-28)
- DATABASE WPI Section Ch, Week 199724 Derwent Publications Ltd., London, GB; Class D13, AN 1997-266451 XP002259691 & JP 09 094060 A (YAKULT HONSHA KK), 8 April 1997 (1997-04-08)

## Description

This invention relates to edible powder materials which have excellent shelf stability of a perfume, or coloring agent contained therein and which, when incorporated into various drinks, foods, cosmetics and the like, can impart a desired fragrance, flavor, color and/or function stably thereto for a long period of time. More particularly, it relates to powder compositions comprising at least one component selected from the group consisting of perfumes, and coloring agents substances, trehalose and water-soluble hemicellulose.

Conventionally, in order to impart a desired fragrance, flavor and/or color to drinks, foods, cosmetics and the like, there have been commonly used powder materials obtained by mixing an oily material selected from oily perfumes, and oil-soluble coloring agents, with a vegetable natural gum solution (e.g., a gum arabic solution) or with an emulsifier (e.g., modified starch or dextrin), an excipient and the like, and then spray-drying the resulting emulsified mixture. Alternatively, such powder materials may also be prepared by mixing or emulsifying an oily material as described above, with a synthetic surface-active agent (e.g., sucrose fatty acid ester, glycerol fatty acid ester or polyglycerol fatty acid ester), a suitable excipient and the like, and then spray-drying the resulting emulsified mixture.

However, when powder materials are prepared by emulsifying a perfume, and coloring agent in the presence of an emulsifier, an excipient and the like, and then drying the resulting emulsified mixture, for example, by spray drying, these powder materials are not always satisfactory from the viewpoint of the shelf stability of the fragrance, flavor, color and/or function.

Meanwhile, in order to improve the shelf stability of perfumes, and coloring agents, the present inventors previously proposed a water-soluble powder perfume obtained by drying an emulsified mixture containing an edible oily material (e.g., perfume) and a water-soluble soybean polysaccharide (see Japanese Patent Laid-Open No. 107937/'95); a method for the preparation of a powdered perfume which comprises drying an emulsified mixture containing a perfume, trehalose, an emulsifier and water (see Japanese Patent Laid-Open No. 107911/'97); and a method for the preparation of a powdered functional substance which comprises drying an emulsified mixture containing a functional substance, trehalose, an emulsifier and water (see Japanese Patent Laid-Open No. 187249/'97).

The above-described propositions are considerably effective in improving the shelf stability of perfumes, coloring agents and functional substances (e.g., vitamins), but are not entirely satisfactory as yet.

The primary object of the present invention is to provide an edible powder composition which has excellent shelf stability of a perfume, and /or coloring agent contained therein and which can be used in various drinks, foods, cosmetics and the like to impart a desired fragrance, flavor, color and/or function stably thereto for a long period of time, without exerting any adverse influence on the inherent fragrance, flavor, color and taste thereof.

The present inventors made an intensive investigation in order to overcome the disadvantages of conventional edible powder materials as described above. As a result, it has now been found that, by emulsifying a perfume, and/or coloring agent while using trehalose (i.e., a nonreducing disaccharide made up of two D-glucose molecules in α,α-1,1 linkage) obtained, for example, by the enzymatic degradation of starch, ins combination with water-soluble hemicellulose, there can be obtained a powder composition having excellent shelf stability of the perfume, and/or coloring agent. Moreover, it has also been found that this powder composition can impart a desired fragrace, flavor, color and/or function stably to various drinks, foods, cosmetics and the like for a long period of time, without exerting any adverse influence on the inherent fragrance, flavor, color and taste thereof. The present invention has been completed on the basis of these findings.

Thus, the present invention provides a powder composition comprising at least one component selected from the group consisting of perfumes, and coloring agents, trehalose and water-soluble hemicellulose as described in claim 1.

The present invention also provides a method for preparing the above-described powder composition which comprises drying an aqueous emulsion containing at least one component selected from the group consisting of perfumes, and coloring agents, trehalose and water-soluble hemicellulose as described in claim 9.

The present invention will be more specifically described hereinbelow.

No particular limitation is placed on the types of the perfumes and coloring agents which can be used as raw materials in the present invention, and there may be used any of the perfumes and coloring agents which are commonly used in drinks, foods, cosmetics and the like. Usable perfumes include, for example, essential oils derived from citrus fruits such as orange, lemon, lime and grapefruit; vegetable essential oils such as flower essential oils, peppermint oil, spearmint oil and spiced oil; powders, extracts, oleo-resins, essences and recovery perfumes derived from cola nuts, coffee, vanilla, cocoa, black tea, green tea, oolong tea and spices; and synthetic perfume compounds, prepared perfume compositions and any mixtures thereof. Usable coloring agents include, for example, α-carotene, β-carotene, lycopene, paprika pigment, annatto pigment, chlorophyll, gardenia pigment, safflower pigment, monascus pigment, beet pigment, elderberry pigment, marigold pigment and cochineal pigment.

The trehalose used in the present invention may be prepared, for example, by culturing yeast in a glucose solution to produce trehalose in yeast cells, and then isolating the trehalose from the yeast cells; or by culturing a bacterium in a glucose solution to produce trehalose in the culture medium, and then separating and recovering the trehalose from the culture medium. However, any commercially available trehalose may also be used. The content of trehalose in the powder composition of the present invention is not strictly limited, but may be suitably chosen according to the type and form of the perfume, coloring agent or functional substance used. However, trehalose is generally used in an amount of 25 to about 85% by weight, and more preferably about 40 to about 80% by weight, based on the total weight of the powder composition.

The water-soluble hemicellulose used in the present invention is hemicellulose which has been made soluble in water by degrading it, for example, by subjecting it to proteolysis with a proteolytic enzyme or by heating it in an aqueous medium under acid conditions. The water-soluble hemicellulose may be derived from cereals and beans, such as soybeans, bean-curd refuse, corn and rice bran. Specific examples thereof include a powder product prepared by providing bean-curd refuse which is obtained as a by-product when bean curd is made from soybeans or when soybean protein is extracted from defatted soybean, homogenizing it with a homogenizer or the like, subjecting it to proteolysis with a protease or hydrolysis in the presence of an acid (for example, hydrolysis at a pH of about 3 to about 7, preferably about 4 to about 5, and a temperature of about 100 to about 150°C, preferably about 110 to about 120°C), separating a water-soluble component by centrifugation or filtrating, and drying it by drying means such as spray drying; and a powder product prepared in the same manner as described above, except that a low-molecular fraction is removed from the water-soluble component. More specifically, there may be used water-soluble hemicellulose derived from a soybean cotyledon, which can be produced by subjecting a soybean cotyledon to heat extraction in an acidic region as described in U.S. Patent 5,700,397 (= EP 0 598 920 B1). Alternatively, commercial products of water-soluble hemicellulose may also be used, and an example thereof is "SOYAFIVE-S" that is sold by Fuji Oil Co., Ltd., Osaka, Japan.

The water-soluble hemicellulose, which may be prepared by any of the above-described methods, contains rhamnose, fucose, arabinose, xylose, galactose, glucose and uronic acid as constituent sugar components, and may further contain mannose and fructose in some cases. Although the contents of these constituent sugar components may vary according to the type of the raw material and the method of preparation, it is usually preferable that the water-soluble hemicellulose used in the present invention contain 1 to 5% by weight of rhamnose, 2 to 8% by weight of fucose, 15 to 50% by weight of arabinose, 4 to 10% by weight of xylose, 25 to 50% by weight of galactose, not greater than 4% by weight of glucose, and 15 to 25% by weight of uronic acid. Moreover, the water-soluble hemicellulose may generally have an average molecular weight of about 50,000 to about 1,000,000, preferably about 100,000 to about 700,000, as measured in a 0. 1M NaNO₃ solution by the limiting viscosity method using standard pullulan.

The content of water-soluble hemicellulose in the powder composition of the present invention is not strictly limited, but may vary according to the type of the perfume, coloring agent or functional substance used, the use of the powder composition of the present invention, and the like. However, water-soluble hemicellulose is used in an amount of 5 to about 60% by weight, and more preferably about 10 to about 40% by weight, based on the total weight of the powder composition.

A powder composition having excellent shelf stability of the perfume, coloring agent and/or functional substance (i.e., vitamin) contained therein is obtained when the weight ratio of trehalose to water-soluble hemicellulose is in the range of 30 : 1 to about 1 : 25, and more preferably about 8 : 1 to about 1 : 1.

The powder composition of the present invention can readily be prepared by mixing at least one component selected from among the above-described perfumes, coloring agents and functional substances, with trehalose, water-soluble hemicellulose and water, and drying the resulting mixture. If necessary, the aforesaid composition may further contain sugars such as sucrose, lactose, glucose, starch syrup and reduced starch syrup; sugar alcohols; various starch hydrolyzates and starch derivatives (e.g., dextrin); starch; gelatin; natural gums such as gum arabic; and the like. The contents of these additives may be suitably chosen according to the properties desired for the powder composition, and the like.

One preferred embodiment of the method for preparing the powder composition of the present invention is as follows. First of all, trehalose and water-soluble hemicellulose as described above are dissolved in water. Then, at least one component selected from among the above-described perfumes, and coloring agents is added thereto and mixed therewith by means of a homomixer, colloid mill, high-pressure homogenizer or the like. The resulting emulsion is dried by drying means such as vacuum drying, spray drying or freeze drying. Thus, there can be obtained a powder composition having excellent shelf stability of the perfume, or coloring agent.

The powder compositions obtained in the above-described manner may be incorporated, in appropriate amounts, into drinks, powdered drinks and foods such as chewing gum, tablet candies, snacks, processed marine products, processed meat products, retort foods, frozen foods, instant noodles and health foods, thus providing drinks and foods to which a desired fragrance, flavor, color and/or function is imparted stably for a long period of time. Moreover, they may be incorporated, in appropriate amounts, into cosmetics such as antiperspirants, shampoos, hair creams, pomades, face powder and lipsticks, thus providing cosmetics to which a desired fragrance, and/or color is imparted stably for a long period of time. Furthermore, they may also be used in sanitary and hygienic materials such as washing detergents, disinfectants and room aromatics; pharmaceutical preparations; tobacco; and the like.

When the powder compositions are incorporated into drinks, foods, cosmetics and the like, their amount used may vary according to the type and form of the product being processed. However, the powder compositions are generally used in an amount of about 0.001 to about 0.1 part by weight, preferably about 0.01 to about 0.05 part by weight, per 1 part by weight of the product being processed.

The present invention is more specifically explained with reference to the following examples, comparative examples and reference examples.

### Example 1

20 g of water-soluble hemicellulose (SOYAFIVE-S LA200, manufactured by Fuji Oil Co., Ltd.; with an average molecular weight of about 200,000) and 60 g of trehalose were added to and dissolved in 100 g of water. This solution was sterilized by heating at 85-90°C for 15 hours. After it was cooled to 40°C, 20 g of a lemon flavor was added thereto and mixed therewith. The resulting mixture was emulsified with a TK-Homomixer (trade name; manufactured by Tokushu Kika Kogyo Co., Ltd.). Using a Mobile Minor type spray dryer (manufactured by Niro Inc.), this emulsion was spray-dried at an inlet temperature of 150°C and an outlet temperature of 80°C to obtain 95 g of a lemon powder perfume (inventive product 1).

### Example 2

The procedure of Example 1 was repeated, except that the amount of trehalose was altered from 60 g to 40 g, and 20 g of gelatin hydrolyzate was additionally used. Thus, there was obtained 95 g of a lemon powder perfume (inventive product 2).

### Comparative Example 1

The procedure of Example 1 was repeated, except that the amount of water was altered from 100 g to 150 g, and 80 g of gum arabic was used in place of 20 g of water-soluble hemicellulose and 60 g of trehalose. Thus, there was obtained 90 g of a lemon powder perfume (comparative product 1).

### Comparative Example 2

The procedure of Example 1 was repeated, except that the amount of water was altered from 100 g to 120 g, and 40 g of gum arabic and 40 g of dextrin (DE10) were used in place of 20 g of water-soluble hemicellulose and 60 g of trehalose. Thus, there was obtained 95 g of a lemon powder perfume (comparative product 2).

### Reference Example 1

According to the procedure described below, tablets were prepared by adding 0.5% of each of the lemon powder perfumes obtained in Examples 1 and 2 and Comparative Examples 1 and 2. The tablets so prepared were subjected to a storage test as described below, and then organoleptically examined for fragrance and flavor by expert panelists. The results thus obtained are shown in Table 1.

**Method for the preparation of tablets**

| (Formulation) | |
|---|---|
| Raw materials | Amount used |
| 1. Powder sugar | 903 g |
| 2. Lactose | 30 |
| 3. Vitamin C | 37 |
| 4. Citric acid powder | 15 |
| 5. 1% aqueous solution of gelatin | 40 |
| 6. Sucrose fatty acid ester | 10 |
| 7. Lemon powder perfume | 5 |
| Total | 1.040 |
| Dry weight | 1,000 |

### (Procedure)

(1) After powder materials 1 to 4 are mixed, material 5 is added thereto and this mixture is agitated until it becomes homogeneous.
(2) The resulting blend is granulated to a size of less than 30 mesh.
(3) The granules are dried at 45°C for 60 minutes.
(4) Materials 6 and 7 are added to and mixed with the granules dried in (3).
(5) The resulting blend is tableted under the following conditions.

| | |
|---|---|
| Weight: | 1.8 g/tablet |
| Diameter: | 2 cm |
| Pressure: | 40 kg/cm²/tablet |

### Storage test method

(1) Tablets were put into a low-density polyethylene bag, which was stored in the dark at 50°C for 4 weeks.
(2) Tablets were put into a high-density polyethylene bag, which was stored under fluorescent lamp illumination at 4,500 lux for 2 weeks.

As a control, tablets were put into an aluminum bag, which was stored at -18°C.

**Table 1**

| Results of Organoleptic Examination | | | |
|---|---|---|---|
| | After storage at -18°C (control) | After storage in the dark at 50°C for 4 weeks | After storage at 4,500 lux for 2 weeks |
| Inventive product 1 | 10 | 9 | 8 |
| Inventive product 2 | 10 | 8 | 7 |
| Comparative product 1 | 10 | 3 | 2 |
| Comparative product 2 | 10 | 2 | 2 |

The numerals given in the above table are relative values obtained by comparing the tested tablets with the control tablets stored at -18°C which are rated as 10. Smaller values indicate that the fragrance and flavor were lose to a higher degree.

As is evident from Table 1, the tablets of Examples 1 and 2 having a powder composition of the present invention incorporated thereinto are superior in the stability of fragrance and flavor to the tablets of Comparative Examples 1 and 2.

### Scanning election micrographs (magnification: ×2000)

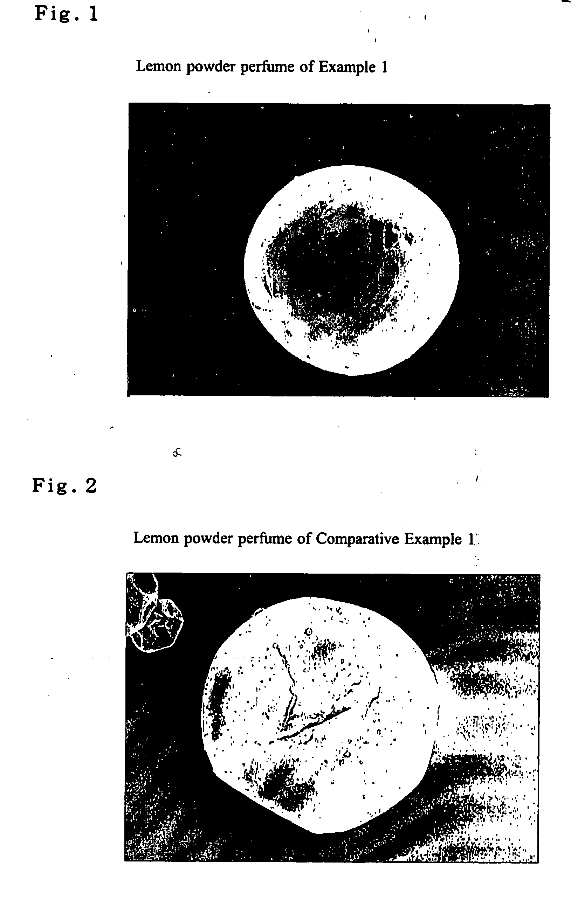

Scanning election micrographs (×2000) of particles of the lemon powder perfumes prepared in Example 1 and Comparative Example 1 are given in FIGs. 1 and 2, respectively. A comparison of FIG. 1 with FIG. 2 reveals that the particle of the lemon powder perfumes of Example 1 in accordance with the present invention is in the form of a dense spherule having a stiff wall surface, and hence exhibits excellent stability of the lemon perfume contained therein.

### Example 3

20 g of water-soluble hemicellulose (SOYAFIVE-S LA200, manufactured by Fuji Oil Co., Ltd.; with an average molecular weight of about 200,000) and 60 g of trehalose were added to and dissolved in 100 g of water. This solution was sterilized by heating at 85-90°C for 15 hours. After it was cooled to 60°C, 20 g of l-menthol was added thereto and mixed therewith. The resulting mixture was emulsified with a TK-Homomixer. Using a Mobile Minor type spray dryer (manufactured by Niro Inc.), this emulsion was spray-dried at an inlet temperature of 160°C and an outlet temperature of 80°C to obtain 95 g of a menthol powder perfume (inventive product 3).

### Example 4

The procedure of Example 3 was repeated, except that the amount of trehalose was altered from 60 g to 40 g, and 20 g of gelatin hydrolyzate was additionally used. Thus, there was obtained 95 g of a menthol powder perfume (inventive product 4).

### Comparative Example 3

The procedure of Example 3 was repeated, except that the amount of water was altered from 100 g to 150 g, and 80 g of gum arabic was used in place of 20 g of water-soluble hemicellulose and 60 g of trehalose. Thus, there was obtained 90 g of a menthol powder perfume (comparative product 3).

### Comparative Example 4

The procedure of Example 3 was repeated, except that the amount of water was altered from 100 g to 120 g, and 40 g of gum arabic and 40 g of dextrin (DE 10) were used in place of 20 g of water-soluble hemicellulose and 60 g of trehalose. Thus, there was obtained 90 g of a menthol powder perfume (comparative product 4).

### Reference Example 2

According to the same procedure as described in Refer-ence Example 1, tablets were prepared by adding 0.5% of each of the menthol powder perfumes obtained in Examples 3 and 4 and Comparative Examples 3 and 4. The tablets so prepared were subjected to a storage test as described below, and then visually examined for the separating-out of menthol. The results thus obtained are shown in Table 2.

### Storage test method

Tablets were put into a low-density polyethylene bag, which was stored in the dark at 50°C for 2, 4 or 12 weeks.

**Table 2**

| Separating-out of Menthol | | | |
|---|---|---|---|
| | 50°C, 2 weeks | 50°C, 4 weeks | 50°C, 12 weeks |
| Example 3 | No | No | No |
| Example 4 | No | No | No |
| Comparative Example 3 | No | Yes | Yes |
| Comparative Example 4 | No | Yes | Yes |

As is evident from Table 2, the tablets of Examples 3 and 4 having a powder composition of the present invention incorporated thereinto are superior in the stability of menthol to the tablets of Comparative Examples 3 and 4.

### Example 5

20 g of water-soluble hemicellulose (SOYAFIVE-S LA200, manufactured by Fuji Oil Co., Ltd.; with an average molecular weight of about 200,000) and 70 g of trehalose were added to and dissolved in 100 g of water. This solution was sterilized by heating at 85-90°C for 15 hours. After it was cooled to 40°C, 10 g of paprika oil was added thereto and mixed therewith. The resulting mixture was emulsified with a TK-Homomixer. Using a Mobile Minor type spray dryer (manufactured by Niro Inc.), this emulsion was spray-dried at an inlet temperature of 160°C and an outlet temperature of 80°C to obtain 95 g of a paprika-containing powder (inventive product 5).

### Comparative Example 5

The procedure of Example 5 was repeated, except that the amount of water was altered from 100 g to 150 g, and 30 g of gum arabic and 60 g of dextrin (DE10) were used in place of 20 g of water-soluble hemicellulose and 70 g of trehalose. Thus, there was obtained 90 g of a paprika-containing powder (comparative product 5).

### Reference Example 3

The paprika-containing powders obtained in Example 5 and Comparative Example 5 were subjected to a storage test as described below. Thereafter, their contents of paprika pigment were measured with a spectrophotometer. The results thus obtained are shown in Table 3.

In Table 3, the retention of paprika pigment after storage at 50°C for 4 weeks is expressed as a percentage based on the content of paprika pigment (= 100%) immediately after preparation.

### Storage test method

Each of the paprika-containing powders (i.e., inventive product 5 and comparative product 5) was put into a low-density polyethylene bag, which was stored in the dark at 50°C for 4 weeks.

**Table 3**

| Retention of Paprika Pigment | |
|---|---|
| | After storage at 50°C for 4 weeks |
| Example 5 | 97.5% |
| Comparative Example 5 | 32.8% |

## Claims

1. A powder composition consisting essentially of (1) at least one component selected from the group consisting of perfumes and coloring agents, (2) 25 to 85 % by weight, based on the total weight of the powder composition, of trehalose and (3) 5 to 60 % by weight, based on the total weight of the powder composition, of water-soluble hemicellulose, the weight ratio of trehalose to water-soluble hemicellulose being in the range of 30:1 to 1:25.

2. The composition of claim 1 wherein the water-soluble hemicellulose is water-soluble hemicellulose derived from a soybean cotyledon.

3. The composition of claim 2 wherein the water-soluble hemicellulose is produced by subjecting a soybean cotyledon to heat extraction in an acidic region.

4. The composition of claim 1 wherein the water-soluble hemicellulose has an average molecular weight in the range of about 50,000 to about 1,000,000.

5. The composition of claim 1 wherein the trehalose is present in an amount of 40 to 80 % by weight based on the total weight of the powder composition.

6. The composition of claim 1 wherein the water-soluble hemicellulose is present in an amount of 10 to 40 % by weight based on the total weight of the powder composition.

7. The composition of claim 1 wherein the weight ratio of trehalose to water-soluble hemicellulose is in the range of 8:1 to 1:1.

8. A method for preparing the powder composition of claim 1 which comprises drying an aqueous emulsion comprising (1) at least one component selected from the group consisting of perfumes and coloring agents, (2) 25 to 85 % by weight, based on the total weight of the powder composition, of trehalose and (3) 5 to 60 % by weight, based on the total weight of the powder composition, of water-soluble hemicellulose, the weight ratio of trehalose to water-soluble hemicellulose being in the range of 30:1 to 1:25.

9. A drink or food containing the composition of claims 1 to 7.

10. A cosmetic containing the composition of claims 1 to 7.

## Patentansprüche

1. Pulverzusammensetzung, im Wesentlichen bestehend aus (1) wenigstens einer Komponente, ausgewählt aus der Gruppe, bestehend aus Riechstoffen und Färbemitteln, (2) 25 bis 85 Gew.-% Trehalose, basierend auf dem Gesamtgewicht der Pulverzusammensetzung und (3) 5 bis 60 Gew.-% wasserlöslicher Hemicellulose, basierend auf dem Gesamtgewicht der Pulverzusammensetzung, wobei das Gewichtsverhältnis von Trehalose zu wasserlöslicher Hemicellulose im Bereich von 30:1 bis 1:25 ist.

2. Zusammensetzung nach Anspruch 1, wobei die wasserlösliche Hemicellulose wasserlösliche Hemicellulose ist, die von einer Sojabohnenkotyledone stammt.

3. Zusammensetzung nach Anspruch 2, wobei die wasserlösliche Hemicellulose hergestellt wird, indem eine Sojabohnenkotyledone einer Hitzeextraktion in einem sauren Bereich unterzogen wird.

4. Zusammensetzung nach Anspruch 1, wobei die wasserlösliche Hemicellulose ein durchschnittliches Molekulargewicht in dem Bereich von etwa 50.000 bis etwa 1.000.000 hat.

5. Zusammensetzung nach Anspruch 1, wobei die Trehalose in einer Menge von 40 bis 80 Gew.-%, basierend auf dem Gesamtgewicht der Pulverzusammensetzung, vorhanden ist.

6. Zusammensetzung nach Anspruch 1, wobei die wasserlösliche Hemicellulose in einer Menge von 10 bis 40 Gew.-%, basierend auf dem Gesamtgewicht der Pulverzusammensetzung, vorhanden ist.

7. Zusammensetzung nach Anspruch 1, wobei das Gewichtsverhältnis von Trehalose zu wasserlöslicher Hemicellulose in dem Bereich von 8:1 bis 1:1 ist.

8. Verfahren zum Herstellen der Pulverzusammensetzung nach Anspruch 1, das das Trocknen einer wässrigen Emulsion umfasst, die (1) wenigstens eine Komponente, ausgewählt aus der Gruppe, bestehend aus Riechstoffen und Färbemitteln, (2) 25 bis 85 Gew.-% Trehalose, basierend auf dem Gesamtgewicht der Pulverzusammensetzung und (3) 5 bis 60 Gew.-% wasserlöslicher Hemicellulose, basierend auf dem Gesamtgewicht der Pulverzusammensetzung, wobei das Gewichtsverhältnis von Trehalose zu wasserlöslicher Hemicellulose im Bereich von 30:1 bis 1:25 ist, umfasst.

9. Getränk oder Nahrungsmittel, das die Zusammensetzung nach den Ansprüchen 1 bis 7 enthält.

10. Kosmetisches Mittel, das die Zusammensetzung nach den Ansprüchen 1 bis 7 enthält.

## Revendications

1. Composition de poudre constituée essentiellement de (1) au moins un composant choisi dans le groupe constitué des parfums et des agents colorants, (2) 25 à 85 % en poids, sur la base du poids total de la composition de poudre, de tréhalose et (3) 5 à 60 % en poids, sur la base du poids total de la composition de poudre, d'hémicellulose hydrosoluble, le rapport en poids du tréhalose sur l'hémicellulose hydrosoluble étant dans la gamme de 30 : 1 à 1 : 25.

2. Composition selon la revendication 1, dans laquelle l'hémicellulose hydrosoluble est une hémi-cellulose hydrosoluble dérivée d'un cotylédon de graine de soja.

3. Composition selon la revendication 2, dans laquelle on produit l'hémicellulose hydrosoluble en effectuant une extraction thermique d'un cotylédon de graine de soja dans une zone acide.

4. Composition selon la revendication 1, dans laquelle l'hémicellulose hydrosoluble a une masse moléculaire moyenne dans la gamme d'environ 50 000 à environ 1 000 000.

5. Composition selon la revendication 1, dans laquelle le tréhalose est présent en une quantité de 40 à 80 % en poids sur la base du poids total de la composition de poudre.

6. Composition selon la revendication 1, dans laquelle l'hémicellulose hydrosoluble est présente en une quantité de 10 à 40 % en poids sur la base du poids total de la composition de poudre.

7. Composition selon la revendication 1, dans laquelle le rapport en poids du tréhalose sur l'hémicellulose hydrosoluble est dans la gamme de 8 : 1 à 1 : 1.

8. Procédé de préparation de la composition de poudre selon la revendication 1, qui comprend le séchage d'une émulsion aqueuse comprenant (1) au moins un composant choisi dans le groupe constitué des parfums et des agents colorants, (2) 25 à 85 % en poids, sur la base du poids total de la composition de poudre, de tréhalose et (3) 5 à 60 % en poids, sur la base du poids total de la composition de poudre, d'hémicellulose hydrosoluble, le rapport en poids du tréhalose sur l'hémicellulose hydrosoluble étant dans la gamme de 30 : 1 à 1 : 25.

9. Boisson ou nourriture contenant la composition selon les revendications 1 à 7.

10. Cosmétique contenant la composition selon les revendications 1 à 7.
